# EUROPÄISCHE PATENTANMELDUNG

(11) **EP 3 909 617 A1**
(43) Veröffentlichungstag der Anmeldung: **17.11.2021**
(21) Anmeldenummer: 21156396.0
(22) Anmeldetag: 10.02.2021
(51) Int. Cl.: A61L 2/10, A47G 1/02, A47G 1/04

(54) **SPIEGEL MIT DESINFEKTIONSEINHEIT**

(30) Priorität: 12.05.2020 DE 102020112817
(71) Anmelder: MIRROR.technology GdbR, 78244 Gottmadingen (DE)
(72) Erfinder: RÖTTCHER, Oliver M, 78269 Volkertshausen (DE); ENGLER, Dovi, 78247 Hilzigen (DE)
(74) Vertreter: Patent- und Rechtsanwälte Behrmann Wagner PartG mbB

(57) **Zusammenfassung**

Die Erfindung betrifft einen Spiegel (1, 1") zur Desinfektion von diesem zugeordneten Oberflächen, aufweisend eine reflektierende Spiegelfläche (S), eine rückseitig der Spiegelfläche (S) angeordnete oder an den Spiegel (1, 1") anordenbare Desinfektionseinheit (2) mit wenigstens einer UVGl-Lichtquelle (8), wobei die Desinfektionseinheit (2) zur Bestrahlung von extern zum Spiegel (1, 1") angeordneten Oberflächen (9) mit UV-Strahlen, insbesondere UV-C Strahlen ausgebildet ist, eine Sensoreinheit (4) zur Erfassung von Umgebungsinformationen und/oder einer Nutzereingabe, und eine Steuerungseinheit (3), welche zur Ansteuerung der Desinfektionseinheit (2) basierend auf von der Sensoreinheit bereitgestellten Daten ausgebildet ist.

## Beschreibung

Die vorliegende Erfindung betrifft einen Spiegel, insbesondere einen Wandspiegel oder ein Spiegelschrank, mit integrierter oder damit verbindbarer, physikalischer Desinfektionseinheit.

Wandspiegel oder Spiegelschränke bzw. Spiegelkabinette sind hinreichend aus dem Stand der Technik bekannt und werden unter anderem in einer Badezimmer- oder Waschraumumgebung in der Regel oberhalb des Waschbeckens installiert. Insbesondere in öffentlichen oder gewerblichen Einrichtungen werden die Waschbecken stark frequentiert und von einer Vielzahl verschiedener Personen in relativ kurz aufeinanderfolgenden Intervallen benutzt. Dadurch können sich auf den Waschbecken und/oder den zugeordneten Armaturen Keime, Bakterien, Viren oder andere Krankheitserreger ansammeln, welche für die nachfolgenden Benutzer ein potentielles Gesundheitsrisiko bergen. Um diesem Risiko zu begegnen, werden diese Einrichtungen in der Regel unter Einsatz eines chemischen Desinfektionsmittels enthaltend beispielsweise Chlordioxid oder Wasserstoffperoxid in regelmäßen Intervallen händisch gereinigt.

Dies birgt jedoch das Problem, dass die für einen wirksamen Infektionsschutz empfohlenen notwendigen Reinigungsintervalle oft nicht eingehalten werden. Zudem besteht das Risiko, dass bei der manuellen Applikation des Desinfektionsmittels Teile der risikobehafteten Oberflächen vergessen oder nicht ausreichend gründlich gereinigt werden, wodurch ein Restrisiko für eine mögliche Kontakt- bzw. Schmierinfektion der nachfolgenden Benutzer bestehen bleibt.

Ebenso sind aus dem Stand der Technik Wandspiegel mit angeordneten Ablageflächen in Form von Unterschränken oder beispielsweise am Spiegel angebrachten Regal- oder Ablageflächen bekannt. Diese werden üblicherweise in Wohn-, Schlafräumen oder Eingangsbereichen verwendet und dienen zur Ablage von bspw. Mobiltelefonen, Schlüsseln, Geldbeuteln, etc. Auch auf diesen Gegenständen können sich durch Gebrauch Keime, Bakterien, Viren oder andere Krankheitserreger ansammeln, welche für die nachfolgenden Benutzer ein potentielles Gesundheitsrisiko bergen.

Aufgabe der vorliegenden Erfindung ist es, basierend auf dem bekannten Stand der Technik, eine verbesserte Spiegelvorrichtung bereitzustellen, welche die vorgenannten Nachteile des Standes der Technik überwindet oder zumindest deutlich abschwächt. Die zugrundeliegende Aufgabe wird durch die Vorrichtung mit den Merkmalen der unabhängigen Ansprüche gelöst. Vorteilhafte Weiterbildungen der Erfindung sind in den Unteransprüchen beschrieben.

In einem ersten Aspekt betrifft die vorliegende Erfindung einen Spiegel zur Desinfektion von diesem zugeordneten Oberflächen, aufweisend eine reflektierende Spiegelfläche, eine rückseitig der Spiegelfläche angeordnete oder an den Spiegel anordenbare Desinfektionseinheit mit wenigstens einer UVGI-Lichtquelle, wobei die Desinfektionseinheit zur Bestrahlung von extern zum Spiegel, beispielsweise unterhalb des Spiegels, angeordneten Oberflächen mit UV-Strahlen, insbesondere UV-C oder vergleichbaren Strahlen, ausgebildet ist, eine Sensoreinheit zur Erfassung von Umgebungsinformationen und/oder einer Nutzereingabe, und eine Steuerungseinheit, welche zur Ansteuerung der Desinfektionseinheit basierend auf von der Sensoreinheit bereitgestellten Daten ausgebildet ist.

Erfindungsgemäß werden dem Spiegel zugeordnete, d.h. sich in der näheren Umgebung des Spiegels befindliche Oberflächen durch die Desinfektionseinheit mittels der ausgestrahlten UVGI-Strahlung automatisch desinfiziert. Durch die Steuerungseinheit kann die Desinfektionseinheit in Abhängigkeit von Bewegungsinformationen und/oder einer Nutzereingabe gesteuert werden, beispielsweise in Abhängigkeit von der Anwesenheit einer Person in der näheren Spiegelumgebung deaktiviert werden und/oder durch eine entsprechende Nutzereingabe selektiv aktiviert werden. Die erfindungsgemäße physikalische Desinfektionseinheit ermöglicht somit eine zuverlässige und wirksame Desinfektion von dem Spiegel zugeordneten Oberflächen, insbesondere gegenüber einer manuellen chemischen Desinfektion aus dem Stand der Technik.

Unter dem Begriff "Desinfektion" wird vorliegend der Prozess der Reduzierung der Anzahl lebensfähiger Mikroorganismen auf einer Oberfläche verstanden. Dies unterscheidet sich von der "Sterilisation", bei der alle Mikroorganismen abgetötet werden, was üblicherweise durch Hitze oder aggressive Chemikalien erzielt wird.

Unter "UVGI-Lichtquelle" wird vorliegend eine Lichtquelle verstanden, welche ultraviolette keimtötende Strahlung zur Inaktivierung von Mikroorganismen emittiert. Hierbei wird insbesondere die DNA/RNA der Mikroorganismen durch die Strahlung zerstört, so dass diese sich nicht reproduzieren können. UV-C-Wellenlängen umfassen Photonen (Lichtteilchen), die im optischen Spektrum am energiereichsten sind (bestehend aus UV, sichtbar und infrarot) und daher photochemisch am aktivsten sind. Die UVGI-Lichtquelle kann somit durch die Verwendung von ultravioletter Strahlungsenergie zur Inaktivierung von Bakterien, Schimmelpilzsporen, Pilzen oder Viren verwendet werden.

In einer bevorzugten Ausführungsform umfasst die UVGI-Lichtquelle UV-C LEDs und UV-A LEDs, vorzugsweise in einem Verhältnis von 2:1, weiter bevorzugt 3:1, am bevorzugtesten von 4:1. Die UV-C LEDs und UV-A LEDs emittieren Licht unterschiedlicher Wellenlängen. Unterschiedliche Wellenlängen wirken auf unterschiedliche Keimarten. Mithin ist die Desinfektionswirkung durch die Verwendung der unterschiedlichen LEDs, nämlich der UV-C LEDs und der UV-A LEDs, weiter verbessert. Ferner emittieren die UV-A LEDs sichtbares Licht. Somit ist es für einen Nutzer sofort sichtbar, wenn die Desinfektionseinheit in Betrieb ist.

Die Desinfektionseinheit ist vorzugsweise rückseitig der Spiegelfläche angeordnet und somit in den Spiegel integriert. Die Desinfektionseinheit kann hierbei auch rückseitig eines nicht-reflektierenden oder teil-reflektierenden Bereichs der Spiegelfläche angeordnet sein. Alternativ kann die Desinfektionseinheit an den Spiegel, beispielsweise in unmittelbarer Umgebung zum Spiegel anordenbar sein. Hierbei kann die Desinfektionseinheit beispielsweise von einem modularen Aufsatzelement umfasst sein, welches an eine Kante, einen Teilbereich oder ein entsprechendes Verbindungselement des Spiegels anbringbar ist. Das modulare Aufsatzelement kann zudem eine Kommunikationsschnittstelle zur Verbindung wenigstens mit der Steuerungseinheit des Spiegels aufweisen und/oder ein Beleuchtungselement zur Raumbeleuchtung, vorzugsweise aufweisend LED oder OLED Elemente umfassen.

In einer bevorzugten Ausführungsform ist die Desinfektionseinheit zur Bestrahlung der Oberflächen eines unterhalb des Spiegels angeordneten Waschbeckens, eines Waschtisches und/oder den diesen zugeordneten Armaturen, sowie generell der Desinfektionseinheit zugeordneten Flächen, wie bspw. auch Unterschränke, Regalflächen oder dergleichen, ausgebildet. Die Ausführungsform kann auch derart ausgestaltet sein, dass die Desinfektionseinheit zur Bestrahlung von Oberflächen oberhalb des Spiegels, innerhalb eines Spiegelschranks, seitlich des Spiegels, oder einer Kombination von mehreren Seiten, wie beispielsweise unterhalb und oberhalb des Spiegels angeordnet bzw. ausgebildet ist. Zur Erreichung einer mehrflächigen Bestrahlung, wie beispielsweise der Bestrahlung von oberhalb, unterhalb und/oder seitlich des Spiegels angeordneten Oberflächen, kann der Spiegel mehrere Desinfektionseinheiten im Spiegel oder am Spiegel aufweisen. Die Desinfektionseinheit weist vorzugsweise eine Mehrzahl von UVGI-LED Chips auf, welche mit einer Wellenlänge zwischen vorzugsweise 200 bis 280nm emittieren. Die Desinfektionseinheit kann hierbei ein LED-Array umfassen, welche sich über wenigstens 25% der Spiegelbreite, vorzugsweise wenigstens 75% der Spiegelbreite erstreckt. Die Desinfektionseinheit ist vorzugsweise zur Emission von UV-C oder vergleichbaren Strahlen ausgebildet. Bei Einsatz mehrerer Desinfektionseinheiten in oder an einem Spiegel können die einzelnen Desinfektionseinheiten auch jeweils eine andere der genannten Strahlungen einsetzen. So könnte eine Desinfektionseinheit, die unten rückseitig im Spiegel integriert ist, mit UV-C-Leuchten zur Flächendesinfektion einer dem Spiegel unterhalb angeordneten Fläche ausgestattet sein, wobei oberhalb am Spiegel gleichzeitig eine weitere Desinfektionseinheit in Form eines Aufsatzelements angeordnet ist, welche auf Flächen oberhalb des Spiegels ausgerichtet ist und beispielsweise zu einer Raum- oder Luft-Desinfektion genutzt werden kann. Die im Spiegel vorhandene Steuerungseinheit ist erfindungsgemäß so ausgestaltet, dass mehrere Desinfektionseinheiten gleichzeitig angesteuert und betrieben werden können, auch wenn diese auf unterschiedlichen Wellenlängen emittieren.

Die zur Desinfektion von Oberflächen notwendige Intensität der UVGI-Lichtquelle ist vorzugsweise auf die Art der Oberfläche und einer gewünschten Sauberkeit ausgerichtet. Die Expositionen variieren hierbei vorzugsweise zwischen 200 und 1.000 J / m2 (20 bis 100 mJ / cm2).

Bei Anwendungen zur Desinfektion der Raumluft, ist die UVGI-Lichtquelle üblicherweise im oder am oberen Bereich des Spiegels angeordnet und so ausgestaltet, dass nur die Luft über 2,1 m Höhe bestrahlt wird.

Die Desinfektionseinheit und/oder die UVGI-Lichtquelle der Desinfektionseinheit weisen vorzugsweise eine gegenüber der Normalen der Spiegelfläche um 10 bis 90°, bevorzugt 40° bis 60° geneigte Hauptabstrahlrichtung auf. Dies bedeutet, dass die Hauptabstrahlrichtung der Desinfektionseinheit gegenüber einer Achse senkrecht zur Spiegelfläche um 10 bis 90°, bevorzugt 40° bis 60° nach unten, oben oder auf die Seite geneigt ist. Die Hauptabstrahlrichtung der Desinfektionseinheit und/oder der UVGI-Lichtquelle der Desinfektionseinheit kann auf beispielsweise 60° begrenzt sein. Hierdurch ist eine maximale Effizienz der emittierten UV-Strahlung erzielbar. In einer besonders bevorzugten Ausführungsform der Erfindung sind die Abstrahlwinkel der im oder am Spiegel angeordneten UVGI-Lichtquellen durch den Benutzer einstellbar oder verstellbar, damit diese effektiv auf die zu bestrahlende Fläche ausgerichtet oder eingestellt werden können.

In einer bevorzugten Ausführungsform umfasst die Desinfektionseinheit ein Gehäuse. Seitenwände des Gehäuses können einen spitzen Winkel, d.h. einen Winkel kleiner 90° einschließen. Besonders bevorzugt beträgt ein Winkel zwischen den Seitenwänden 55°. Hierdurch sind Nutzer vor einer direkten Bestrahlung durch die UVGI-Lichtquelle geschützt. Die Desinfektionseinheit kann mit einer Quarzglasabdeckung versehen sein. Dies erhöht die UV-Strahlungsübertagung der Desinfektionseinheit. Zudem sind die UVGI-Lichtquellen geschützt. In einer besonders bevorzugten Ausführungsform der Erfindung umfasst die Desinfektionseinheit UV-beständige versiegelte elektronische Steckverbinder. Somit wird die Lebensdauer der Desinfektionseinheit verlängert.

Die von der Sensoreinheit erfassbaren Umgebungsinformationen umfassen vorzugsweise wenigstens die Anwesenheit und/oder Abwesenheit einer Person bzw. eines Benutzers des Spiegels insbesondere in unmittelbarer Nähe des Spiegels und weiter bevorzugt innerhalb eines Abstrahlbereichs der Desinfektionseinheit. Die Sensoreinheit kann hierbei einen Näherungssensor, Bewegungssensor und/oder einen Kamerasensor aufweisen. Diese können zur Anwesenheitserkennung einer Person vor dem Spiegel und insbesondere innerhalb eines Abstrahlbereichs der Desinfektionseinheit ausgebildet sein. Die von der Sensoreinheit erfassten Umgebungsinformationen können zudem auch eine Umgebungstemperatur und/oder Feuchtigkeit umfassen. Die Sensoreinheit kann zusätzlich entsprechende Sensorik aufweisen. Die Erfassungsparameter der Sensoreinheit sind vorzugsweise durch den Benutzer des Spiegels einstellbar; beispielsweise über eine zugeordnete oder zuordenbare Steuerungsapp.

In einer bevorzugten Ausführungsform umfasst die von der Sensoreinheit erfassbare Nutzereingabe wenigstens eine manuelle Aktivierung und/oder Deaktivierung der Desinfektionseinheit. Hierbei kann die Sensoreinheit beispielsweise wenigstens einen manuellen Bedienknopf wie beispielsweise einen in die Spiegelfläche integrierten Touchsensor oder einen im Gehäuse des Spiegels integrierten Schalter aufweisen. Die Sensoreinheit kann zudem von einer unten näher beschriebenen insbesondere internen Bedieneinheit umfasst sein oder mit dieser zur Erfassung von Bedien- oder Steuerbefehlen verbunden sein.

Die Steuerungseinheit kann zur drahtgebundenen und/oder drahtlosen Kommunikation mit einer externer Datenbankeinheit, einer internen Bedieneinheit, insbesondere zur berührungsempfindlichen oder berührungslosen Steuerung, und/oder einer externen Bedieneinheit, insbesondere einer Fernbedienung und/oder einem Smartphone oder Tablet mit entsprechender Steuerungsapp, ausgebildet sein. Hierfür kann die Steuerungseinheit eine Kommunikationsschnittstelle, umfassend beispielsweise eine WiFi-, eine Mobilfunk-, eine WLAN-, eine LAN- oder eine Bluetooth-Schnittstelle, aufweisen.

In einer bevorzugten Ausführungsform ist die Steuerungseinheit ausgebildet, die Desinfektionseinheit bei Anwesenheitserkennung einer Person durch die Sensoreinheit, insbesondere in unmittelbarer Nähe zum Spiegel und/oder innerhalb eines Abstrahlbereichs der Desinfektionseinheit, zu deaktivieren. Hierbei kann die Steuerungseinheit einen von einem Benutzer beispielsweise mittels externer Softwaremittel adaptierbaren räumlichen Erkennungsbereich aufweisen, in welchem die entsprechende Anwesenheitserkennung erfolgt. Hierdurch wird eine unerwünschte Aktivierung der Desinfektionseinheit bei Anwesenheit eines Benutzers verhindert.

In einer bevorzugten Ausführungsform ist die Steuerungseinheit ausgebildet, die Desinfektionseinheit basierend auf intern gespeicherten und/oder extern zugeführten Steuerungsdaten in vordefinierten Zeitintervallen und/oder für einen vordefinierten Zeitraum zu aktivieren. Auf diese Weise können vordefinierte Reinigungspläne beispielsweise abhängig vom Benutzeraufkommen definiert und in der Steuerungseinheit hinterlegt werden. Die entsprechenden Steuerungsdaten können in einer integrierten Speichereinheit der Steuerungseinheit gespeichert sein. Die Steuerungsdaten können der Steuerungseinheit zudem mittels einer Kommunikationsschnittstelle des Spiegels von einer externen Datenbankeinheit und/oder einer externen Bedieneinheit zur Verfügung gestellt werden. Letztere kann zudem zur individuellen Anpassung der Steuerungsdaten ausgebildet sein. Die externe Bedieneinheit kann beispielsweise ein an den Spiegel mittels der Kommunikationsschnittstelle verbindbares Smartphone oder Tablet mit entsprechender Steuerungsapp sein. Im Falle einer Erkennung einer Person in unmittelbarer Umgebung des Spiegels wie oben beschrieben erfolgt vorzugsweise auch bei dieser Ausführungsform eine Deaktivierung der Desinfektionseinheit, vorzugsweise wenigstens für die Dauer der Anwesenheitserkennung durch die Sensoreinheit.

In einer bevorzugten Ausführungsform ist der Spiegel als Spiegelschrank ausgebildet, wobei der Spiegel wenigstens eine zweite Desinfektionseinheit mit wenigstens einer UVGI-Lichtquelle aufweist, welche zur Bestrahlung von im Inneren des Spiegelschranks angeordneten Oberflächen, insbesondere von Regalböden, Ablageflächen und/oder Toilettenartikeln ausgebildet ist.

Die zweiten Desinfektionseinheit bzw. die UVGI-Lichtquelle weist einen gegenüber der Normalen der Spiegelfläche vorzugsweise um 70 bis 90° geneigte Hauptabstrahlrichtung auf. Besonders bevorzugt ist die zweite Desinfektionseinheit an einer obersten Innenfläche bzw. Innenwand im Spiegelschrank angeordnet und derart, dass diese im Wesentlichen vertikal nach unten in den Innenraum des Spiegelschranks abstrahlt. Es hat sich als besonders effektiv herausgestellt, wenn die Rück- und Innenwände innerhalb des Spiegelschranks mit einem hoch reflektierenden Material versehen sind, sodass diese als Reflektor für die UVGI-Lichtquelle dienen. So wird sichergestellt, dass im Spiegelschrank vorhandene Flächen oder Gegenstände von allen Seiten angestrahlt bzw. desinfiziert werden können.

Der Spiegel bzw. Spiegelschrank weist vorzugsweise einen Positionssensor zum Erfassen einer Position einer Spiegelschranktüre auf. Diese ist vorzugsweise mit der Steuerungseinheit verbunden und gibt Rückmeldung, ob der Spiegelschrank geöffnet oder geschlossen ist. Die Steuerungseinheit ist hierbei vorzugsweise zur Ansteuerung der zweiten Desinfektionseinheit basierend auf den von dem Positionssensor bereitgestellten Daten ausgebildet.

In einer bevorzugten Ausführungsform weist der Spiegel wenigstens ein rückseitig der Spiegelfläche und vorzugsweise in einem nicht-reflektierenden oder teil-reflektierenden Bereich der Spiegelfläche angeordnetes Beleuchtungselement zur Raumbeleuchtung auf. Das Beleuchtungselement kann insbesondere LED- oder OLED-Leuchtmittel aufweisen. Das Beleuchtungselement ist vorzugsweise zur nutzerspezifischen Einstellung von Farbtemperatur und Leuchtintensität (Dimm-Funktion) ausgebildet. Eine Bedienung des Beleuchtungselements kann durch im Spiegel integrierte oder am Spiegel vorgesehene Bedienelemente oder durch ein mit dem Spiegel verbundenes und beispielsweise an der Wand angeordnetes Bedienpanel erfolgen. In einer bevorzugten Ausbildungsform ist das Beleuchtungselement derart angesteuert, dass dieses bei Erkennung eines Benutzers in der Nähe des Spiegels automatisch aktiviert wird.

Der Spiegel kann zudem optische, visuelle und/oder akustische Ausgabemittel aufweisen, vorzugsweise umfassend eine in der Spiegelfläche integrierte visuelle Betriebsanzeige und/oder einen rückseitig der Spiegelfläche angeordneten Flächenlautsprecher.

Die Desinfektionseinheit und/oder die wenigstens eine UVGI-Lichtquelle der Desinfektionseinheit strahlt im Betrieb vorzugsweise mit einer spezifischen Farbe aus, beispielsweise in blau, damit der Benutzer neben der visuellen Betriebsanzeige sofort erkennen kann, wann die Desinfektion in Betrieb ist. Hierfür kann die Desinfektionseinheit und/oder die UVGI-Lichtquelle neben einer beispielsweise UV-C Strahlung emittierenden Lichtquelle eine weitere, rein farbliches Licht emittierende Lichtquelle wie beispielsweise eine blaues oder rotes Licht emittierende LED Lichtquelle aufweisen.

In einer bevorzugten Ausführungsform umfassen die optischen bzw. visuellen Ausgabemittel ein Display bzw. einen Flachbildschirm zur Darstellung von Informationen. Das Display kann dabei mit den vorgenannten akustischen Ausgabemitteln, insbesondere einem Flächenlautsprecher, verbunden sein. Die Leuchtstärke des Displays beträgt beispielsweise mindestens 280 cd/m². Die Diagonale des Displays ist vorzugsweise mindestens 5 Zoll. Bevorzugt sind die Ausgabemittel zu Servicezwecken auswechselbar bzw. abnehmbar ausgebildet.

Die visuelle Betriebsanzeige und/oder das Display sind/ist beispielsweise an der Rückseite der Spiegelfläche hinter mindestens einem nicht-reflektierenden oder teil-reflektierenden Bereich zur Darstellung von Informationen angeordnet bzw. hinterlegt. Der nicht-reflektierende bzw. teil-reflektierende Bereich kann innerhalb der reflektierenden Spiegelfläche ausgebildet sein. Ein Transmissionsgrad des nicht-reflektierenden oder teil-reflektierenden Bereichs beträgt vorzugsweise mindestens 50%, mehr bevorzugt mindestens 80%, am bevorzugtesten mindestens 90%.

In einer bevorzugten Ausführungsform beträgt der Reflexionsgrad des Spiegels mindestens 50% und der Transmissionsgrad des nicht-reflektierenden oder teil-reflektierenden Bereichs beträgt mindestens 50%. Somit sind/ist die visuelle Betriebsanzeige und/oder das Display, die/das hinter dem nicht-reflektierenden bzw. teil-reflektierenden Bereich angeordnet sind/ist, nicht sichtbar, wenn die visuelle Betriebsanzeige bzw. das Display ausgeschalten ist.

Die Ausgabemittel sind vorzugsweise mit der Steuerungseinheit verbunden und sind von dieser ansteuerbar. Die Steuerungseinheit kann zur drahtgebundenen und/oder drahtlosen Kommunikation mit dem Internet und/oder einem Netzwerk verbunden sein. Über das Internet und/oder das Netzwerk sind zumindest Teile der auf dem/den Ausgabemittel(n) dargestellten Informationen beziehbar. Hierfür kann die Steuerungseinheit eine Kommunikationsschnittstelle, umfassend beispielsweise eine WiFi-, eine Mobilfunk-, eine WLAN-, eine LAN- oder eine Bluetooth-Schnittstelle, aufweisen.

In einer bevorzugten Ausführungsform ist das Display mit einem Content-Management-System (CMS) zur Einspielung bzw. Übertragung weiterer Informationen verbunden. Bei den weiteren Informationen kann es sich um Wetter-, Verkehrsinformationen, Werbe-, Videoclips und/oder Bildanzeigen handeln. Vorzugsweise ist das Display in verschiedene Bereiche aufgeteilt bzw. untergliedert, auf welchen unterschiedliche Informationen dargestellt sind.

Der Spiegel besteht vorzugsweise aus Glas, welches auf seiner Rückseite beschichtet ist. Der Reflexionsgrad des Spiegels ist vorzugsweise mindestens 50%, mehr bevorzugt mindestens 75% und am bevorzugtesten mindestens 85%. Ein Transmissionsgrad des nicht-reflektierenden oder teil-reflektierenden Bereichs beträgt vorzugsweise mindestens 50%, mehr bevorzugt mindestens 80%, am bevorzugtesten mindestens 90%. In einer bevorzugten Ausführungsform beträgt der Reflexionsgrad des Spiegels mindestens 50% und der Transmissionsgrad des nicht-reflektierenden oder teil-reflektierenden Bereichs beträgt mindestens 50%.In einer bevorzugten Ausführungsform ist der Spiegel wenigstens spritzwassergeschützt ausgebildet, vorzugsweise nach der Schutzklasse IP24 oder einer vergleichbaren Schutzklasse. Der Spiegel kann eine Heizeinrichtung zur Beheizung der Spiegelfläche aufweisen. Die Heizeinrichtung kann beispielsweise eine Heizfolie an einer Rückseite der Spiegelfläche sein, mit der ein Beschlagen des Spiegels in einem Feuchtraum oder Bad verhindert werden kann. Der Spiegel weist vorzugsweise eine Befestigungsvorrichtung oder mindestens ein Rahmenprofil zur Wandmontage auf.

In einer bevorzugten Ausführungsform ist die Steuerungseinheit ausgebildet, die Ausgabemittel und die Desinfektionseinheit basierend auf intern gespeicherten und/oder extern zugeführten Steuerungsdaten zu steuern. Die entsprechenden Steuerungsdaten können in der integrierten Speichereinheit der Steuerungseinheit gespeichert sein. Die Steuerungsdaten können der Steuerungseinheit zudem mittels der Kommunikationsschnittstelle des Spiegels von einer Datenbankeinheit und/oder einer Bedieneinheit zur Verfügung bzw. bereitgestellt gestellt werden. Letztere kann zudem zur individuellen Anpassung der Steuerungsdaten ausgebildet sein. Mittels der Bedieneinheit ist beispielsweise eine Strahlungsintensität der Desinfektionseinheit regelbar bzw. einstellbar.

Die Bedieneinheit kann hierbei die zuvor beschriebene Sensoreinheit zur Erfassung einer Nutzereingabe umfassen oder von dieser mitumfasst sein. Die Bedieneinheit kann beispielsweise zur berührungsempfindlichen oder berührungslosen Steuerung ausgebildet sein. Die Bedieneinheit kann weiterhin eine Fernbedienung mit einer entsprechenden Steuerungsapp, ein Smartphone mit einer entsprechenden Steuerungsapp und/oder ein Tablet mit einer entsprechenden Steuerungsapp umfassen.

Für die berührungsempfindliche Steuerung umfasst die Bedieneinheit vorzugsweise ein kapazitives Steuerelement. Die entsprechende Bedieneinheit und/oder die zugeordnete Sensoreinheit kann bzw. können hierbei an der Spiegelvorderseite angeordnet sein. Im Falle einer Erkennung eines Steuerbefehls bzw. einer Nutzereingabe sind/ist die Ausgabemittel und/oder die Desinfektionseinheit steuerbar.

In einer bevorzugten Ausführungsform ist die berührungslose Steuerung als kapazitive Steuerung ausgebildet. Die entsprechende Bedieneinheit kann ein vorzugsweise an der Spiegelvorderseite angeordnetes berührungsloses Steuerelement umfassen. Das Steuerelement ist vorzugsweise zur Ansteuerung mittels holographischer Projektion, insbesondere an bzw. vor der Spiegelvorderseite, ausgebildet. Hierbei kann ein Nutzer die Ausgabemittel und/oder die Desinfektionseinheit berührungslos und somit besonders hygienisch steuern.

Die berührungslose Bedieneinheit umfasst bevorzugt Steuerungssymbole, eine Lichtquelle und eine entsprechend ausgebildete Sensoreinheit. Die Steuerungssymbole können Aussparungen von auf der Spiegelrückseite bzw. Glasrückseite angeordneten Folien sein. Auch können die Steuerungssymbole in der Spiegelfläche bzw. Glasfläche auf Nano-Ebene ausgebildet sein. Beispielsweise kann der Spiegel bzw. die Spiegelfläche eine spezielle Glasbeschichtung aufweisen, in welche Darstellungen von Tasten oder Steuersymbolen eingearbeitet sind, die mit einer entsprechend zugeordneten Lichtquelle beleuchtet werden, wodurch holographische Bilder vor dem Spiegel für einen Nutzer sichtbar "in der Luft schweben". Die Lichtquelle ist vorzugsweise an einer Seitenfläche des Spiegels angeordnet bzw. vorgesehen und ausgebildet, Licht seitlich in die Glasfläche einzustrahlen bzw. einzukoppeln. Die Sensoreinheit umfasst hierbei vorzugsweise einen optischen Sensor, welcher bevorzugt im Spiegel bzw. Glas eingebettet oder auf der Glasrückseite angeordnet bzw. fixiert ist. Der optische Sensor ist vorzugsweise derart ausgebildet, dass dieser eine Berührung der an bzw. vor der Spiegelfläche projizierten Bilder durch einem Nutzer erkennt bzw. detektiert, und hierbei identifiziert, welches der Steuerungssymbole von dem Nutzer berührt bzw. selektiert wurde, und die ermittelten Informationen an die Steuerungseinheit überträgt.

Bei der erfindungsgemäßen Verwendung des Spiegels wird der erfindungsgemäße Spiegel in einem Feuchtraum, insbesondere Bad, Toilette oder dergleichen, oder in einem Eingangsbereich des Hauses oder Wohn- und Schlafräumen verwendet, wobei der Spiegel an einer Wand des Raums fest fixiert ist. Die Verwendung des Spiegels in einem Feuchtraum wird insbesondere dadurch möglich, dass der Spiegel spritzwassergeschützt ausgebildet ist.

In einem weiteren Aspekt betrifft die Erfindung ein System aufweisend einen erfindungsgemäßen Spiegel, wie vorgehen beschrieben, und ein dem Spiegel zugeordnetes, insbesondere in Wirkreichweite der Desinfektionseinheit angeordnetes Einrichtungsstück, Möbelstück und/oder eine Stellvorrichtung, aufweisend wenigstens eine zu desinfizierende Oberfläche. Das Einrichtungsstück ist vorzugsweise ein sanitäres Einrichtungsstück wie beispielsweise ein Waschbecken oder ein Waschtisch. Das Möbelstück ist vorzugsweise ein Regal, ein Tisch oder eine Ablage. Die Stellvorrichtung ist vorzugsweise eine Armatur, insbesondere eine Waschtisch- oder Waschbeckenarmatur.

Weitere Vorteile, Merkmale und Einzelheiten der Erfindung ergeben sich aus der nachfolgenden Beschreibung bevorzugter Ausführungsbeispiele sowie anhand der Zeichnungen, diese zeigen in:
- **Fig. 1**: eine schematische Vorderansicht eines erfindungsgemäßen Spiegels mit integrierter Desinfektionseinheit;
- **Fig. 2**: eine schematische Darstellung des Spiegels gemäß Fig. 1 mit den integrierten Komponenten;
- **Fig. 3a-c**: drei bevorzugte Ausführungsformen des erfindungsgemäßen Spiegels gemäß Fig. 1;
- **Fig. 4**: eine schematische Vorderansicht eines weiteren erfindungsgemäßen Spiegels mit integrierter Desinfektionseinheit;
- **Fig. 5**: eine schematische Darstellung des Spiegels gemäß Fig. 4 mit den integrierten Komponenten; und
- **Fig. 6**: eine schematische Darstellung der Desinfektionseinheit des erfindungsgemäßen Spiegels gemäß Fig. 4.

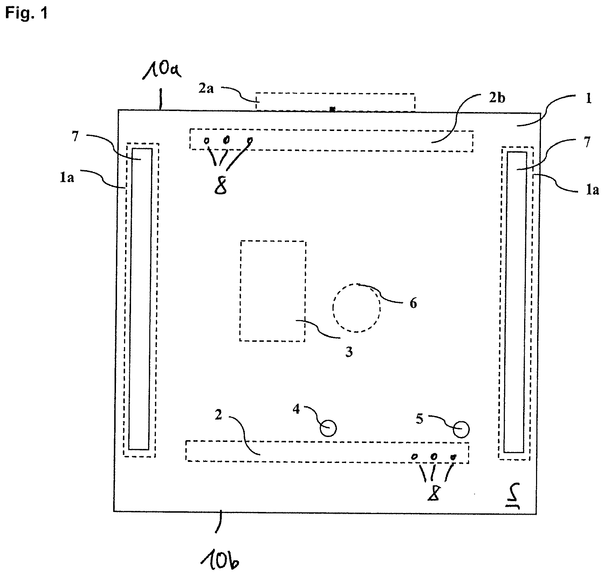

**Fig. 1** zeigt einen Spiegel 1, insbesondere einen Wandspiegel oder einen Spiegelschrank, aufweisend eine vorzugsweise hinsichtlich der Reflexionseigenschaften homogen ausgebildete, reflektierende Spiegelfläche S. Diese kann wenigstens eine, vorzugsweise zwei nicht-reflektierende oder teil-reflektierende Bereiche 1a aufweisen, in welchen jeweils eine Beleuchtungseinheit 7 zur Raumbeleuchtung hinterlegt bzw. auf der Rückseite der Spiegelfläche S angeordnet ist. Die Beleuchtung kann mittels direkter oder indirekter Beleuchtung ausgeführt sein. Bei einer direkten Beleuchtung ist die Beleuchtungseinheit 7 frontal hinter den nicht-reflektierende oder teil-reflektierende Bereichen 1a angebracht; bei einer indirekten Beleuchtung ist die Beleuchtungseinheit 7 seitlich neben den nicht-reflektierende oder teil-reflektierende Bereichen 1a angebracht. Bei einer Ausführungsform mit direkter Beleuchtung hat sich als vorteilhaft herausgestellt, wenn zwischen Beleuchtungseinheit 7 und den nicht-reflektierende oder teil-reflektierende Bereichen 1a ein Diffusor vorhanden ist, damit die einzelnen Lichtpunkte der Beleuchtungseinheit 7 nicht zu sehen sind. Durch Verwendung des Diffusors entsteht somit eine homogene Ausleuchtung ohne Wahrnehmung einzelner Lichtspots.

Der Spiegel 1 weist wenigstens eine Desinfektionseinheit 2 auf, welche rückseitig der Spiegelfläche S angeordnet und somit in den Spiegel 1 integriert sein kann. Die Desinfektionseinheit 2 weist wenigstens ein, mehr bevorzugt eine Vielzahl von UVGI-Lichtquellen 8, vorzugsweise UVGI-LED Chips, besonders bevorzugt in der Form eines LED-Arrays auf. Die Desinfektionseinheit 2 kann hierbei auch rückseitig eines nicht-reflektierenden oder teil-reflektierenden Bereichs der Spiegelfläche S angeordnet sein. Die Desinfektionseinheit 2 ist vorzugsweise in einem unteren Bereich, nahe einer Unterkante 10b des Spiegels 1 angeordnet.

Alternativ oder zusätzlich kann die Desinfektionseinheit 2 an den Spiegel 1, beispielsweise in unmittelbarer Umgebung zum Spiegel, anordenbar sein. Wie in Fig. 1 gezeigt, kann die Desinfektionseinheit 2a beispielsweise in einem modularen Aufsatzelement für den Spiegel 1 integriert sein oder als solches ausgebildet sein. Das Aufsatzelement bzw. die Desinfektionseinheit 2a kann beispielsweise an eine Oberkante 10a des Spiegels 1 oder in unmittelbarer Nähe zur Oberkante 10a anbringbar sein. Hierbei kann das Aufsatzelement eine Kommunikationsschnittstelle 18 zur Anbindung an den Spiegel 1 und/oder eine Beleuchtungseinheit 19 zur Raumbeleuchtung aufweisen.

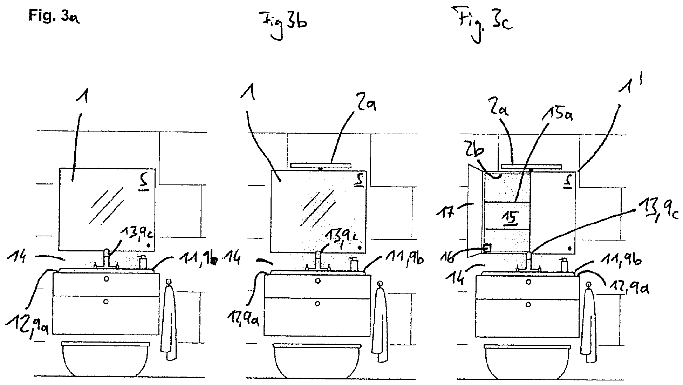

Die jeweilige Desinfektionseinheit 2, 2a ist vorzugsweise zur Bestrahlung von unterhalb des Spiegels angeordneten Oberflächen ausgebildet. Die Desinfektionseinheit 2, 2a erstreckt sich hierbei über wenigstens 25%, mehr bevorzugt über wenigstens 75% der Breite des Spiegels 1. Alternativ oder zusätzlich kann die jeweilige Desinfektionseinheit 2, 2a oder eine zusätzliche Desinfektionseinheit (nicht gezeigt) auch zur Bestrahlung von Oberflächen seitlich und/oder oberhalb des Spiegels angeordnet bzw. ausgebildet sein. Hierbei kann eine jeweilige Desinfektionseinheit insbesondere auch zur Raum- oder Luft-Desinfektion genutzt werden. Der Spiegel 1 kann zudem als Spiegelschrank 1' ausgebildet sein. Hierbei weist der Spiegelschrank 1' vorzugsweise eine zweite bzw. weitere Desinfektionseinheit 2b auf. Diese ist zur Bestrahlung von im Inneren 15 des Spiegelschranks angeordneten Oberflächen 15a, beispielsweise Regalböden, und/oder darauf angeordneten Gegenständen ausgebildet (vgl. Fig. 3), vorzugsweise insbesondere wenn der Spiegelschrank geschlossen ist und/oder keine Person in unmittelbarer Nähe zum Spiegel steht.

Der Spiegel 1 umfasst weiterhin eine Sensoreinheit 4 zur Erfassung von Umgebungsinformationen und/oder einer Nutzereingabe. Diese kann insbesondere einen Näherungs- oder Bewegungssensor umfassen, welcher die Anwesenheit einer Person in unmittelbarer Nähe zum bzw. vor dem Spiegel erkennt. Die Sensoreinheit 4 kann zudem wenigstens ein manuelles Bedienelement zur manuellen Aktivierung oder Deaktivierung der Desinfektionseinheit 2 aufweisen.

Der Spiegel 1 umfasst zudem eine Steuerungseinheit 3, welche zur Ansteuerung der Desinfektionseinheit 2 basierend auf von der Sensoreinheit 4 bereitgestellten Daten ausgebildet ist. Bevorzugt deaktiviert die Steuerungseinheit 3 die Desinfektionseinheit 2 bzw. setzt deren Betrieb aus, wenn die Sensoreinheit 4 die Anwesenheit eines Benutzers feststellt.

Der Spiegel 1 kann zudem optische und/oder visuelle Ausgabemittel aufweisen, vorzugsweise umfassend eine in der Spiegelfläche integrierte visuelle Betriebsanzeige 5, beispielsweise eine LED-Statusanzeige, und/oder einen rückseitig der Spiegelfläche S angeordneten Flächenlautsprecher 6. Die Ausgabemittel sind mit der Steuerungseinheit 3 verbunden und können von dieser angesteuert werden.

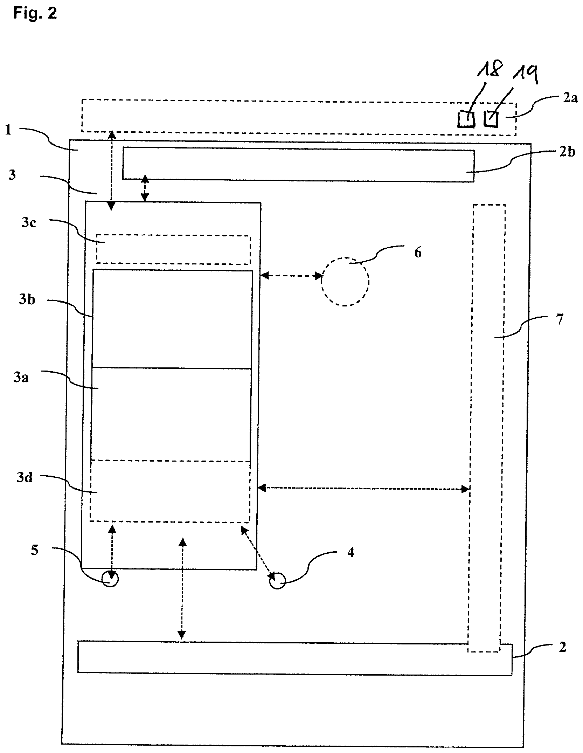

Wie in **Fig. 2** gezeigt, ist die Steuerungseinheit 3 mit den vorgesehenen Desinfektionseinheiten 2, 2a, 2b der Sensoreinheit 4 sowie den optischen bzw. visuellen und/oder akustischen Ausgabemitteln 5, 6 verbunden. Die Kommunikationsschnittstelle 18 der an den Spiegel 1 anordenbaren Desinfektionseinheit 2a kann hierbei vorzugsweise zur drahtgebundenen und/oder drahtlosen Kommunikation mit der Steuerungseinheit 3 des Spiegels 1 ausgebildet sein.

Die Steuerungseinheit 3 weist vorzugsweise ein erstes Steuermodul 3a auf, welches die Ansteuerung der Desinfektionseinheit 2, 2a, 2b in Abhängigkeit von den zugeführten Daten der Sensoreinheit 4 regelt bzw. steuert. Dies erfolgt vorzugsweise basierend auf einer Anwesenheitserfassung einer Person in unmittelbarer Nähe zum Spiegel. Alternativ oder zusätzlich kann eine Regelung basierend auf einer vordefinierten zeitlichen Ansteuerung, insbesondere gemäß einem hinterlegten Reinigungsplan, erfolgen. Das Steuermodul 3a kann hierbei integrierte Speichermittel aufweisen, in welchen Parameter für eine Aktivierung der Desinfektionseinheit 2, 2a, 2b in vordefinierten Zeitintervallen und/oder für einen vordefinierten Zeitraum hinterlegt sind.

Die Steuerungseinheit 3 weist vorzugsweise ein zweites Ausgabemodul 3b auf, welches die Ausgabe über die optischen und/oder visuellen Ausgabemitteln 5, 6 steuert. Beispielsweise kann bei der Aktivierung und/oder Deaktivierung der jeweiligen Desinfektionseinheit 2, 2a, 2b ein entsprechendes optisches oder visuelles Signal an einen Benutzer ausgegeben werden. Über die visuellen Ausgabemittel 5 wie beispielsweise eine Status-LED kann der jeweilige Status der Desinfektionseinheit 2, 2a, 2b als "aktiv" oder "inaktiv" beispielsweise als grün oder rot ausgegeben werden.

Die Steuerungseinheit 3 weist vorzugsweise weiterhin ein drittes Kommunikationsmodul bzw. eine Kommunikationsschnittstelle 3c auf, welche zur drahtgebundenen und/oder drahtlosen Kommunikation mit einer externer Datenbankeinheit und/oder einer externen Bedieneinheit, insbesondere einer Fernbedienung und/oder einem Smartphone oder Tablet mit entsprechender Steuerungsapp, ausgebildet ist. Diese kann insbesondere eine WiFi-, eine Mobilfunk-, eine WLAN-, eine LAN- oder eine Bluetooth-Schnittstelle umfassen. Über eine derartige externe Datenbankanbindung oder mittels einer entsprechenden Bedieneinheit können bspw. automatische Desinfektionszyklen der Desinfektionseinheiten 2 ,2a, 2b definiert werden, die dann zu den definierten Zeiten aktiviert werden. Im Falle einer Anwesenheitserkennung durch die Sensoreinheit 4 des Spiegels 1 wird der jeweilige automatische Desinfektionszyklus wenigstens für die Dauer der Anwesenheit der Person vorzugsweise unterbrochen.

Die Steuerungseinheit 3 weist vorzugsweise weiterhin ein viertes Beleuchtungssteuerungsmodul 3d zur Ansteuerung der Beleuchtungseinheit 7 auf. Das Beleuchtungssteuerungsmodul 3d kann zur manuellen Ansteuerung der Beleuchtungseinheit 7 mittels im oder am Spiegel 1 vorgesehener manueller Bedienelemente oder zur automatischen Ansteuerung ausgebildet sein. Das Beleuchtungssteuerungsmodul 3d ist vorzugsweise zur nutzerspezifischen Einstellung von Farbtemperatur und Leuchtintensität der Beleuchtungseinheit 7 ausgebildet. Das Beleuchtungssteuerungsmodul 3d kann zudem die Beleuchtungseinheit 7 basierend auf zugeführten Sensordaten, beispielsweise durch die Sensoreinheit 4, derart ansteuern, dass bei Erkennung eines Benutzers in der Nähe des Spiegels 1 eine automatische Aktivierung der Beleuchtungseinheit 7 erfolgt.

**Fig. 3a-3c** zeigen bevorzugte Ausführungsformen des erfindungsgemäßen Spiegels 1, 1'.

**Fig. 3a** zeigt hierbei den Spiegel 1 mit integrierter Desinfektionseinheit 2 (hier nicht dargestellt) im unteren Bereich des Spiegels. Die Desinfektionseinheit 2 weist eine gegenüber der Normalen der Spiegelfläche S um 10° bis 90°, bevorzugt 40° bis 60° geneigte Hauptabstrahlrichtung auf und ist somit nach unten in Richtung unterhalb des Spiegels angeordneten Oberflächen ausgerichtet. Ein Abstrahlbereich 14 der Desinfektionseinheit 2 ist vorzugsweise derart ausgerichtet, dass dieser auf die Oberflächen 9a, 9b, 9c eines unterhalb des Spiegels angeordneten Waschbeckens 11, eines Waschtisches 12 und/oder den diesen zugeordneten Armaturen 13 ausgebildet ist.

**Fig. 3b** zeigt eine alternative Ausführungsform, in welcher die Desinfektionseinheit 2 an einer Oberkante 10a des Spiegels 1 angeordnet bzw. anordenbar ist. Hierbei ist die Desinfektionseinheit 2 bezüglich ihres Abstrahlbereichs 14 derart angepasst bzw. ausgebildet, dass dieser ebenfalls auf die unterhalb des Spiegels angeordneten Oberflächen 9a, 9b, 9c eines Waschbeckens 11, eines Waschtisches 12 und/oder den diesen zugeordneten Armaturen 13 ausgerichtet ist.

**Fig. 3c** zeigt eine dritte Ausführungsform, in welcher der Spiegel als Spiegelschrank 1' ausgebildet ist. Hierbei weist der Spiegelschrank zusätzlich eine zweite Desinfektionseinheit 2b auf, welche zur Bestrahlung von im Inneren 15 des Spiegelschranks angeordneten Oberflächen 15a, insbesondere von Regalböden und/oder darauf angeordneten Gegenständen ausgebildet ist. Die zweiten Desinfektionseinheit 2b weist einen gegenüber der Normalen der Spiegelfläche vorzugsweise um 70° bis 90° geneigte Hauptabstrahlrichtung auf und ist besonders bevorzugt an einer obersten Innenfläche bzw. Innenwand im Spiegelschrank 1' angeordnet. Der Spiegelschrank weist zudem einen Positionssensor 16 zum Erfassen einer Position der Spiegelschranktüren 17 auf. Dieser ist vorzugsweise mit der Steuerungseinheit 3 verbunden. Diese ist vorzugsweise derart ausgebildet, dass bei Öffnung der Schranktüren die Desinfektionseinheit 2b deaktiviert bzw. ein laufender Desinfektionszyklus für die Dauer der Öffnung unterbrochen wird.

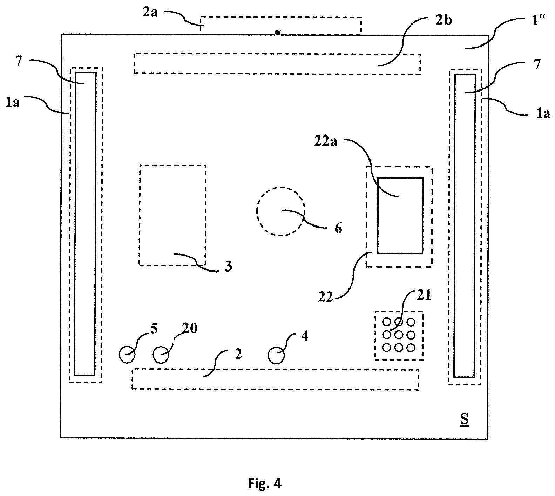

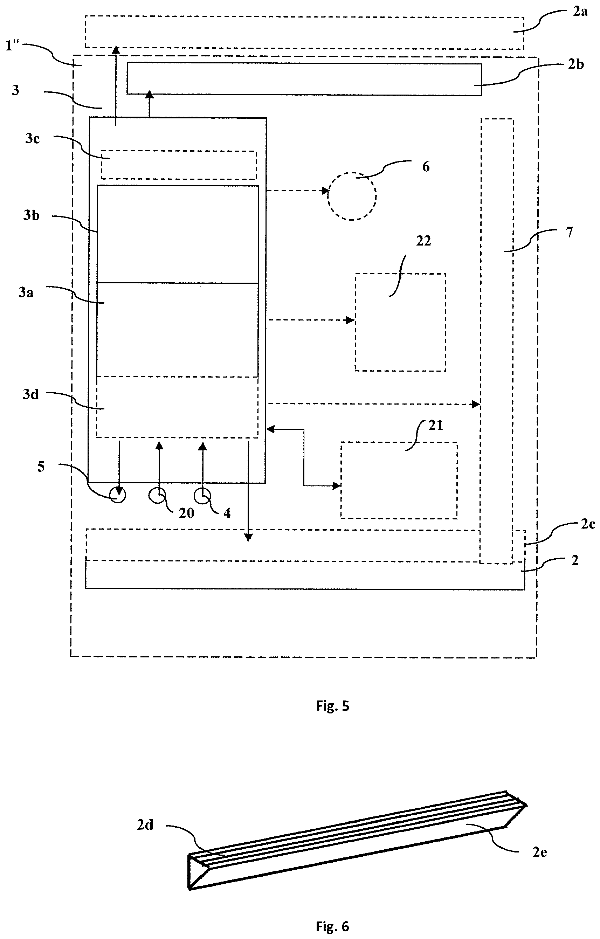

**Fig. 4** und **5** zeigen eine weitere Ausführungsform eines Spiegels 1" nach der Erfindung. Der Spiegel 1" entspricht dem Spiegel 1, der in den Fig. 1 und 2 dargestellt ist, und umfasst zusätzlich folgende Elemente:

Der Spiegel 1" umfasst neben der visuellen Betriebsanzeige 5 und dem Flächenlautsprecher 6 weitere optische bzw. visuelle und/oder akustische Ausgabemittel. Vorzugsweise umfasst der Spiegel 1" eine Status-LED 2c und ein Display 22 bzw. einen Flachbildschirm zur Darstellung von Informationen und/oder des jeweiligen Status der Desinfektionseinheit 2, 2a, 2b.

Die Status-LED 2c und/oder das Display 22 sind/ist beispielsweise mit der Steuerungseinheit 3 verbunden und können/kann von dieser angesteuert werden. Insbesondere ist die Ausgabe über die Status-LED 2c bzw. das Display 22 mittels des zweiten Ausgabemoduls 3b der Steuerungseinheit 3 steuerbar. Beispielsweise kann über die Status-LED 2c und/oder das Display 22 bei der Aktivierung und/oder Deaktivierung der jeweiligen Desinfektionseinheit 2, 2a, 2b ein entsprechendes optisches bzw. visuelles Signal an einen Benutzer ausgegeben werden.

Über das Display 22 können weitere Informationen wie Wetter-, Verkehrsinformationen, Werbe-, Videoclips und/oder Bildanzeigen angezeigt bzw. dargestellt werden. Zum Empfangen dieser Informationen können/kann das Display 22 und/oder die Steuerungseinheit 3 mit einem Content-Management-System (CMS) verbunden sein. Das Display 22 ist beispielsweise in verschiedene Bereiche aufgeteilt bzw. untergliedert. In den verschiedenen Bereichen können unterschiedliche Informationen dargestellt sein.

Das Display 22 ist beispielsweise an der Rückseite der Spiegelfläche S hinter mindestens einem nicht-reflektierenden oder teil-reflektierenden Bereich 22a angeordnet bzw. hinterlegt. Das Display 22 kann in dem Spiegel 1" integriert sein. Bevorzugt ist das Display 22 derart in dem Spiegel 1" integriert, dass er zu Servicezwecken auswechselbar bzw. abnehmbar ist.

Der Spiegel 1" umfasst vorzugsweise wenigstens eine Bedieneinheit in Form eines berührungsempfindlichen kapazitiven Steuerelements 20 und/oder eines berührungslosen kapazitiven Steuerelements 21. Diese können mit der Steuerungseinheit 3, insbesondere der Kommunikationsschnittstelle 3c der Steuerungseinheit 3 verbunden sein und mit dieser kommunizieren. Die ebenfalls dargestellte Sensoreinheit 4 zur Erfassung einer Nutzereingabe kann hierbei abweichend von der Darstellung in den Figuren auch von einer Bedieneinheit 20, 21 mitumfasst sein. Die dargestellte Sensoreinheit 4 kann alternativ oder zusätzlich zur Erfassung von Umgebungsinformationen ausgebildet sein.

**Fig. 6** zeigt eine bevorzugte Ausführungsformen der Desinfektionseinheit 2, 2a, 2b des erfindungsgemäßen Spiegels 1, 1',1".

Wie in **Fig. 6** gezeigt, umfasst die Desinfektionseinheit 2, 2a, 2b vorzugsweise ein Gehäuse. Das Gehäuse kann einen V-förmigen Querschnitt aufweisen. Seitenwände des Gehäuses können einen spitzen Winkel, d.h. einen Winkel kleiner 90°, einschließen. Besonders bevorzugt beträgt ein Winkel zwischen den Seitenwänden des Gehäuses 55°. Hierdurch sind Nutzer vor direkter Bestrahlung durch die UVGI-Lichtquelle geschützt. An der offenen Seite des Gehäuses bzw. der Vorderseite der Desinfektionseinheit 2, 2a, 2b ist ein Aluminiumprofil 2d zur Wärmeableitungsoptimierung angeordnet. Die Desinfektionseinheit 2, 2a, 2b, insbesondere ihr Gehäuse kann mit einer Quarzglasabdeckung 2e versehen sein. Hierdurch ist eine UV-Strahlungsübertragung maximiert und die UVGI-Lichtquellen werden geschützt. Bevorzugt umfasst die Desinfektionseinheit 2, 2a, 2b UV-beständige versiegelte elektronische Steckverbinder.

Die oben beschriebenen Ausführungsformen sind lediglich beispielhaft, wobei die Erfindung keineswegs auf die in den Figuren gezeigten Ausführungsformen beschränkt ist.

### Bezugszeichenliste

- 1,1": Spiegel
- 1': Spiegelschrank
- 1a: nicht-reflektierend oder teil-reflektierender Bereich
- 2: Desinfektionseinheit
- 2a: Desinfektionseinheit als Aufsatzelement
- 2b: zweite Desinfektionseinheit (Spiegelschrank)
- 2c: Status-LED
- 2d: Aluminiumprofil zur Wärmeableitungsoptimierung
- 2e: Quarzglasabdeckung
- 3: Steuerungseinheit
- 3a: Steuermodul
- 3b: Ausgabemodul
- 3c: Kommunikationsmodul
- 3d: Beleuchtungssteuerungsmodul
- 4: Sensoreinheit
- 5: visuelle Ausgabemittel
- 6: Audioausgabemittel
- 7: Beleuchtungseinheit
- 8: UVGI Lichtquellen
- 9: Oberflächen (zu desinfizieren)
- 9a: Waschtischoberfläche
- 9b: Waschbeckenoberfläche
- 9c: Armaturoberfläche
- 10a: Spiegeloberkante
- 10b: Spiegelunterkante
- 11: Waschbecken
- 12: Waschtisch
- 13: Armatur(en)
- 14: Abstrahlbereich
- 15: Innenraum Spiegelschrank
- 15a: Oberflächen Innenraum/Regalböden
- 16: Positionssensor
- 17: Spiegelschranktüre
- 18: Kommunikationsschnittstelle
- 19: Beleuchtungselement
- 20: (kapazitives) berührungsempfindliches Steuerelement
- 21: (kapazitives) berührungsloses Steuerelement
- 22: Display
- 22a: nicht-reflektierend oder teil-reflektierender Bereich
- S: Spiegelfläche

## Patentansprüche

1. Spiegel (1, 1") zur Desinfektion von diesem zugeordneten Oberflächen, aufweisend
eine reflektierende Spiegelfläche (S),
eine rückseitig der Spiegelfläche (S) angeordnete oder an den Spiegel (1, 1") anordenbare Desinfektionseinheit (2) mit wenigstens einer UVGI-Lichtquelle (8), wobei die Desinfektionseinheit (2) zur Bestrahlung von extern zum Spiegel (1, 1") angeordneten Oberflächen (9) mit UV-Strahlen, insbesondere UV-C Strahlen ausgebildet ist,
eine Sensoreinheit (4) zur Erfassung von Umgebungsinformationen und/oder einer Nutzereingabe, und
eine Steuerungseinheit (3), welche zur Ansteuerung der Desinfektionseinheit (2) basierend auf von der Sensoreinheit bereitgestellten Daten ausgebildet ist.

2. Spiegel nach Anspruch 1,
wobei die Desinfektionseinheit (2) zur Bestrahlung von seitlich, unterhalb und/oder oberhalb des Spiegels (1, 1") angeordneten Oberflächen (9), vorzugsweise eines unterhalb des Spiegels angeordneten Waschbeckens (11), eines Waschtisches (12) und/oder den diesen zugeordneten Armaturen (13), ausgebildet ist.

3. Spiegel nach Anspruch 1 oder 2,
wobei der Spiegel (1, 1") wenigstens eine weitere Desinfektionseinheit aufweist, welche zur Bestrahlung einer seitlich und/oder oberhalb des Spiegels (1, 1") angeordneten Oberfläche, insbesondere auch zur Raum- oder Luft-Desinfektion aufweist.

4. Spiegel nach einem der vorhergehenden Ansprüche,
wobei die Desinfektionseinheit (2) eine Mehrzahl von UV-C LED Chips aufweist, welche Strahlung vorzugsweise mit einer Wellenlänge zwischen 200 bis 280nm emittieren, und/oder wobei die UVGI-Lichtquelle (8) eine gegenüber der Normalen der Spiegelfläche um 10 bis 90°, bevorzugt 40° bis 60° geneigte Hauptabstrahlrichtung aufweist.

5. Spiegel nach einem der vorhergehenden Ansprüche,
wobei die Desinfektionseinheit (2, 2a, 2b) und/oder die UVGI-Lichtquelle (8) hinsichtlich einer Hautabstrahlrichtung einstellbar am Spiegel (1, 1") angeordnet ist.

6. Spiegel nach einem der vorhergehenden Ansprüche,
wobei die Sensoreinheit (4) einen Näherungssensor, Bewegungssensor und/oder einen Kamerasensor umfasst, welcher zur Anwesenheitserkennung einer Person vor dem Spiegel (1, 1") und insbesondere innerhalb eines Abstrahlbereichs (14) der Desinfektionseinheit (2) ausgebildet ist.

7. Spiegel nach einem der vorhergehenden Ansprüche,
wobei die Sensoreinheit (4) zur Erfassung einer Nutzereingabe, insbesondere zur manuellen Aktivierung und/oder Deaktivierung der Desinfektionseinheit (2) ausgebildet ist.

8. Spiegel nach einem der vorhergehenden Ansprüche,
wobei die Steuerungseinheit (3) ausgebildet ist, die Desinfektionseinheit (2) bei Anwesenheitserkennung einer Person durch die Sensoreinheit (4) zu deaktivieren, und/oder
wobei die Steuerungseinheit (3) ausgebildet ist, die Desinfektionseinheit (2) basierend auf intern gespeicherten und/oder extern zugeführten Steuerungsdaten in vordefinierten Zeitintervallen und/oder für einen vordefinierten Zeitraum zu aktivieren.

9. Spiegel nach einem der vorhergehenden Ansprüche,
wobei der Spiegel (1, 1") als Spiegelschrank (1') ausgebildet ist und der Spiegel wenigstens eine zweite Desinfektionseinheit (2b) aufweist, welche zur Bestrahlung von im Inneren (15) des Spiegelschranks angeordneten Oberflächen (15a), insbesondere Regalböden, ausgebildet ist und wobei der Spiegel (1') vorzugsweise einen Positionssensor (16) zum Erfassen einer Position der Spiegelschranktüren (17) umfasst und die Steuerungseinheit (3) zur Ansteuerung der zweiten Desinfektionseinheit (2b) basierend auf von dem Positionssensor (16) bereitgestellten Daten ausgebildet ist.

10. Spiegel nach einem der vorhergehenden Ansprüche,
wobei der Spiegel (1, 1") optische bzw. visuelle und/oder akustische Ausgabemittel aufweist, vorzugsweise umfassend eine in der Spiegelfläche integrierte visuelle Betriebsanzeige (5), einen rückseitig der Spiegelfläche (S) angeordneten Flächenlautsprecher (6) und/oder ein Display (22) zur Darstellung von Informationen, welches an der Rückseite der Spiegelfläche (S) hinter einem nicht-reflektierenden oder teil-reflektierenden Bereich der Spiegelfläche (S) angeordnet ist.

11. Spiegel nach Anspruch 10,
wobei die Steuerungseinheit (3) ausgebildet, die Ausgabemittel (5, 6, 22) und die Desinfektionseinheit (2) basierend auf intern gespeicherten und/oder extern zugeführten, insbesondere über eine Kommunikationsschnittstelle bereitgestellten, Steuerungsdaten zu steuern.

12. Spiegel nach einem der vorhergehenden Ansprüche,
wobei die Steuerungseinheit (3) zur drahtgebundenen und/oder drahtlosen Kommunikation mit einer externer Datenbankeinheit, einer internen Bedieneinheit, insbesondere zur berührungsempfindlichen oder berührungslosen Steuerung, und/oder einer externen Bedieneinheit, insbesondere einer Fernbedienung und/oder einem Smartphone, ausgebildet ist.

13. Spiegel nach Anspruch 12,
wobei die interne Bedieneinheit ein vorzugsweise an einer Spiegelvorderseite angeordnetes berührungsloses Steuerelement (21) umfasst, welches zur Ansteuerung mittels holographischer Projektion insbesondere an bzw. vor der Spiegelvorderseite ausgebildet ist.

14. Spiegel nach einem der vorhergehenden Ansprüche,
wobei die an den Spiegel (1, 1") anordenbare Desinfektionseinheit (2b) als modulares Aufsatzelement ausgebildet ist, aufweisend eine Kommunikationsschnittstelle (18) zur Anbindung an die Steuerungseinheit (3) des Spiegels (1, 1").

15. Verwendung eines Spiegels (1, 1") nach einem der vorhergehenden Ansprüche in einem Feuchtraum, insbesondere Bad, Toilette oder dergleichen, wobei der Spiegel (1, 1") an einer Wand des Feuchtraums über einem Waschbecken (11) und/oder einem Waschtisch (12) mit zu desinfizierenden Oberflächen (9a, 9b) fest fixiert ist.
